# FASCICULE DE BREVET EUROPEEN

(11) **EP 0 752 829 B1**
(45) Date de publication et mention de la délivrance du brevet: **05.08.1998**
(21) Numéro de dépôt: 95915226.5
(22) Date de dépôt: 29.03.1995
(51) Int. Cl.: A61B 10/00

(54) **DISPOSITIF A USAGE UNIQUE DE RECUEIL, ET EVENTUELLEMENT D'ANALYSE, D'UN LIQUIDE CORPOREL**
WEGWERFVORRICHTUNG ZUR PROBENENTNAHME UND GEGEBENENFALLS ANALYSE VON KÖRPERFLÜSSIGKEIT
DISPOSABLE DEVICE FOR COLLECTING AND OPTIONALLY ANALYSING A BODY FLUID

(30) Priorité: 29.03.1994 FR 9404066
(43) Date de publication de la demande: 15.01.1997
(73) Titulaire: Chaffringeon, Bernard, 1025 Saint-Sulpice (CH)
(72) Inventeur: Chaffringeon, Bernard, 1025 Saint-Sulpice (CH)
(74) Mandataire: Guerre, Dominique
(86) Numéro de dépôt international: FR9500402
(87) Numéro de publication internationale: WO9526161

(56) Documents cités:
- EP-A- 0 101 981
- WO-A-92/21774
- DE-A- 3 327 444
- DE-A- 3 327 967
- US-A- 5 231 992

## Description

La présente invention concerne le recueil, et éventuellement l'analyse d'un liquide corporel, directement dans une cavité intracorporelle allongée, et à titre d'exemple non limitatif, la présente invention sera introduite, définie et expliquée par rapport au recueil de la glaire cervicale dans la cavité vaginale de la femme pour la détection de la période fertile.

Afin de déterminer les périodes de fertilité de la femme, il est connu et il a été proposé de détecter et suivre la présence et/ou concentration en certains constituants biochimiques ou biologiques de ladite glaire, tels qu'une peroxydase ou un composé présentant une activité peroxydasique, ou tels qu'un mucopolysaccharide ou une glycoprotéine, par l'utilisation de réactifs ou systèmes réactifs colorés, par exemple, dans le premier cas un composé d'oxydoréduction, dont au moins la forme oxydée est colorée, par exemple le gaïacol, et dans le second cas la safranine.

Pour la mise en oeuvre de tels réactifs ou systèmes réactionnels, on a généralement proposé des solutions sommaires, et en pratique difficilement mises en oeuvre par la femme, à savoir :
- le prélèvement in situ de la glaire cervicale, par un élément susceptible de recueillir la glaire, tel qu'un écouvillon, puis la mise en contact dudit élément, à l'extérieur de la cavité vaginale, avec le réactif sous forme liquide;
- l'introduction dans la cavité vaginale d'un tampon absorbant. imprégné avec le réactif, et l'extraction dudit tampon au bout d'un certain temps, avec observation de la couleur ou du changement de couleur du réactif sur l'absorbant.

Toutes ces solutions relativement sommaires ne sont pas adaptées à une utilisation banalisée, cohérente avec les pratiques d'hygiène intime de la femme.

S'agissant du prélèvement de liquides ou sécrétions corporelles, indépendamment de leur analyse biochimique ou biologique, postérieure ou concomitante au prélèvement et en schématisant, deux types de solutions sont couramment envisagés.

La première consiste à recueillir extracorporellement lesdits liquides ou sécrétions, à l'endroit même de leur excrétion, ou au voisinage dudit endroit, sur tout support ou moyen de recueil approprié. Une telle solution est décrite dans le document DE-A-33 27 444, qui décrit un dispositif de recueil d'urine, muni de lèvres pour entourer de manière étanche le méat urinaire féminin, et comprenant un moyen de recueil du liquide corporel et un moyen anti-retour pour empêcher des fuites du liquide recueilli hors du moyen de recueil. Cette solution reste toutefois inadaptée lorsque le liquide ou sécrétion demeure pour l'essentiel à l'intérieur du corps, telle la glaire cervicale à l'intérieur du vagin.

La seconde consiste à recueillir intracorporellement lesdits liquides ou sécrétions, dans la cavité intracorporelle même. Ceci suppose l'utilisation de dispositifs ou moyens de recueil adaptés à être introduits, maintenus et extraits dans la cavité intracorporelle. Aujourd'hui, les dispositifs commercialisés ou décrits par la littérature disponible, sont essentiellement adaptés au recueil de cellules ou de prélèvement cellulaires.

La présente invention a pour objet un dispositif à usage unique, de type intracorporel, spécifiquement adapté au recueil de liquides ou fluides corporels.

Le dispositif selon l'invention comprend de manière générale,un moyen de collecte d'un liquide corporel, monté ou intégré à dans le dispositif, s'étendant selon la longueur de ce dernier, et un moyen anti-retour du liquide corporel collecté, fermant le moyen de collecte lorsque ledit liquide s'écoule dans le dispositif, dans le sens opposé à celui de la collecte et, en outre
- un élément de conduit relativement rigide, adapté en forme et dimensions, pour être logé et retenu directement au contact de la paroi intérieure d'une cavité intracorporelle allongée, présentant une première extrémité ouverte, dite col, et une seconde extrémité fermée, dite fond; cet élément de conduit est suffisamment raide selon sa longueur pour être poussé, par tout moyen approprié, y compris manuellement, par son fond, et ainsi introduit par son col dans la cavité intracorporelle de recueil ; et l'agencement ou les matériaux de la surface extérieure de l'élément de conduit sont choisis pour être ou demeurer biocompatibles avec la paroi intérieure de la cavité intracorporelle;
- le moyen de collecte du liquide corporel, est monté ou intégré à l'intérieur de l'élément de conduit, et s'étend selon la longueur de ce dernier, d'une entrée de section transversale relativement importante, en vis-à-vis ou au niveau du col dudit élément de conduit, à une sortie de section transversale relativement faible, en vis-à-vis ou en face du fond du même élément de conduit;
- le moyen anti-retour du liquide corporel collecté ferme la sortie de section transversale relativement faible du moyen de collecte précité, lorsque le liquide collecté s'écoule dans ledit élément de conduit, dans le sens opposé à celui de la collecte.

Par "biocompatible", on entend le fait que le contact entre la surface extérieure de l'élément de conduit et la paroi de la cavité intracorporelle ne génère aucune réaction biologique adverse, de type toxique ou allergique par exemple.

Dans le cas d'un dispositif selon l'invention, adapte au recueil de la glaire cervicale dans la cavité vaginale, on aboutit à une solution requérant pour sa mise en oeuvre les mêmes gestes que ceux nécessaires à l'hygiène intime de la femme.

Préférentiellement, mais lorsque le dispositif de recueil sert, en même temps et directement, de dispositif réactionnel, un moyen réactionnel est disposé à l'intérieur de l'élément de conduit, du côté de son fond, en vis-à-vis de la sortie du moyen de collecte, et comprend au moins un réactif, par exemple déposé sur une couche absorbante, susceptible de réagir avec au moins un composant du liquide corporel recueilli, pour donner au moins un produit de réaction, notamment coloré, révélant la présence dudit composant dans ledit liquide corporel.

Préférentiellement dans ce cas, le fond de l'élément de conduit est transparent, et le réactif est disposé contre ce fond, et ceci en vis-à-vis de la sortie du moyen de collecte, de telle sorte que lorsque le produit de réaction est coloré, cette couleur ou absence de couleur peut être vue ou visualisée par l'utilisateur ou l'utilisatrice.

Le dispositif de recueil selon l'invention, décrit précédemment, peut donc être utilisé directement, en particulier sans ajout ultérieur de réactif ou révélateur coloré par l'utilisateur ou l'utilisatrice, et dans ce dernier cas, l'observation de la réaction coloré est immédiate après le retrait du dispositif.

La présente invention est maintenant décrite par référence au dessin annexé, dans lequel :
- la figure 1 représente une vue en coupe d'un dispositif à usage unique selon l'invention;
- la figure 2 représente un ensemble prêt à l'emploi, comprenant un dispositif selon figure 1 avec un système applicateur.

De manière générale, un dispositif 1 à usage unique, de recueil du liquide corporel, par exemple de glaire cervicale, directement dans une cavité intracorporelle allongée, par exemple la cavité vaginale, comprend :
- un élément 2 de conduit, adapté en forme et dimensions pour être logé et retenu au contact de la paroi intérieure, par simple constriction, de la cavité intracorporelle, présentant une extrémité ouverte ou col 2a et une extrémité fermée ou fond 2b; cet élément est en soi suffisamment raide ou relativement rigide selon sa longueur, pour être poussé par son fond 2b, et introduit par son col 2a dans la cavité intracorporelle; et de manière générale, comme décrit ci-après, sa surface extérieure est choisie pour être biocompatible avec la paroi intérieure de la cavité intracorporelle ;
- un moyen de collecte 3 du liquide corporel, monté de manière amovible, ou intégré à l'intérieur de l'élément de conduit 2, s'étendant selon la longueur de ce dernier, d'une entrée 3a de section transversale relativement importante, au niveau du col 2a de l'élément de conduit 2, à une sortie 3b de section transversale relativement faible, en face mais à distance du fond 2b de l'élément de conduit 2 ;
- un moyen 4 anti-retour du liquide corporel collecté, fermant la sortie 3b du moyen de collecte lorsque le liquide collecté s'écoule dans l'élément de conduit 2, dans le sens opposé à celui de la collecte ;

L'élément de conduit 2 peut avoir une structure composite et comprendre un tube 5 relativement rigide, par exemple en matière plastique transparente, avec un col 5a et un fond 5b, sur lequel est éventuellement emmanché un manchon 6 biocompatible, entourant au moins la paroi latérale du tube, constitué par exemple par un tube extérieur 6a en composés d'origine naturelle tels que la cellulose ou la ouate, et une matrice intérieure 6b rigide de support en polymère pour ledit deuxième tube, dont la bordure antérieure (dans le sens de l'introduction du dispositif) est arrondie, et dont la bordure postérieure est à bord droit.

Le moyen de collecte 3 avec son moyen anti-retour 4 sont montés de manière amovible, ou fixe, par rapport à l'élément de conduit 2, et plus précisément, au tube rigide 5, grâce à un pas de vis 5e et un épaulement 5d prévus sur le tube 5, au niveau de son col 5a.

Le moyen anti-retour 4 comprend un dispositif de clapet 7, monté de manière mobile ou sur support ajouré par rapport à la sortie 3b du moyen de collecte 3, rappelé contre cette sortie dans le sens opposé à celui de la collecte du fluide corporel, le tout étant disposé dans un collecteur 8 dudit fluide, en face du fond 2b de l'élément de conduit 2.

Un lien d'extraction 15, sous la forme d'un fil ou cordon est fixé à l'élément de conduit 2, du côté du fond 2b, par l'intermédiaire d'une gorge 5c prévue dans le tube 5.

Un moyen ou système réactionnel 9 est disposé à l'intérieur de l'élément de conduit 2, du côté de son fond 2b, en vis-à-vis de la sortie 3b du moyen de collecte 3, et incorpore au moins un réactif, et éventuellement un agent fluidifiant pour la gare cervicale, déposé par exemple soit directement soit sur une couche absorbante 12, susceptible de réagir avec au moins un composant du liquide corporel collecté, pour donner au moins un produit de réaction coloré, révélant la présence dudit composant dans le liquide corporel. Comme déjà dit, le réactif coloré sur son support absorbant, est disposé contre le fond 2b, en vis-à-vis de la sortie 3b du moyen de collecte 3, et plus précisément contre la paroi de fond transparente 10 du tube 5. La couche imprégnée du réactif 9 est recouverte par un voile 11 hemi-perméable, en ce sens qu'il est perméable vis-à-vis du liquide corporel collecté, dans le sens de la collecte, mais qu'il retient ce même liquide dans l'autre sens. Ce voile peut également être imprégné de l'agent fluidifiant pour la glaire cervicale, dans le cas où le dispositif servirait à analyser celle-ci, ledit agent favorisant le passage des composés que l'on souhaite détecter et étant par exemple un agent tensioactif.

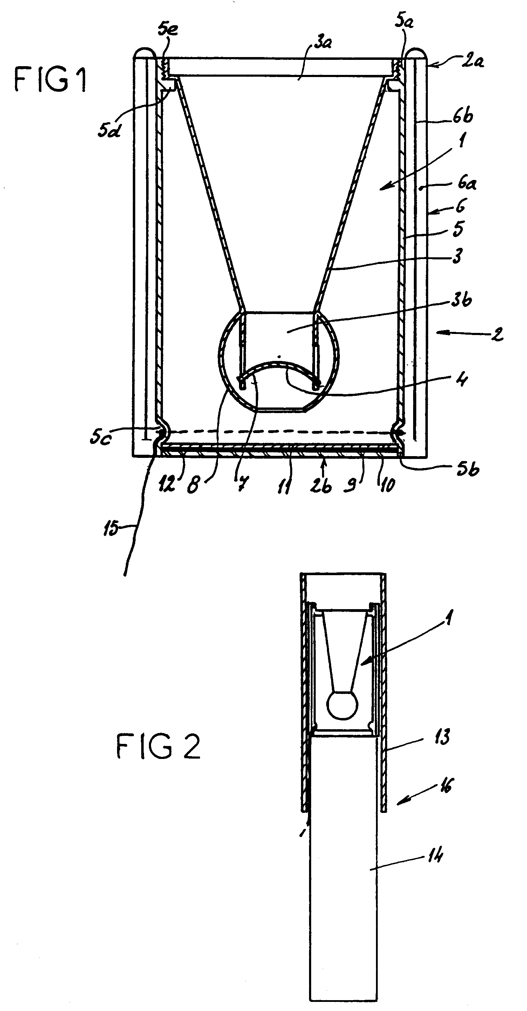

De manière générale, mais comme non représenté à la figure 1, la forme et les dimensions de l'élément 2 de conduit sont adaptées à celles de la cavité vaginale.

Conformément à la figure 2, le dispositif précédemment décrit peut faire partie d'un ensemble prêt à l'emploi comprenant ce même dispositif 1 et un système applicateur 16. Ce dernier comprend un tube-guide 13, à l'intérieur duquel le dispositif 1 de recueil est inséré, et un poussoir 14 logé à l'intérieur du tube-guide, en butée contre le dispositif de recueil. Un tel système peut être introduit directement dans la cavité intracorporelle, par exemple la cavité vaginale, et en poussant le poussoir, on libère le dispositif de recueil par rapport au système applicateur, pour le mettre en place dans cette même cavité.

Le dispositif de recueil peut être utilisé, non seulement pour la détection d'un composant dans un liquide corporel, mais aussi pour détecter toute condition chimique, biochimique ou biologique de ce même liquide corporel, à des fins de diagnostic, prophylactiques ou thérapeutiques. Ceci signifie que ce dispositif de recueil peut incorporer des réactifs ou systèmes réactionnels très différents, de type purement chimique, par exemple enzymatique, ou biologique, par exemple un antigène ou un anticorps.

En conséquence, un dispositif de recueil selon l'invention peut recevoir de très larges applications, au rang desquelles on peut citer :
- la détection d'une hormone, et notamment d'un pic hormonal
- le prélèvement et l'analyse histologique et/ou cytochimique d'un liquide corporel, notamment pour détecter des états pathologiques, ou mettre en évidence certaines périodes physiologiques d'un cycle naturel, par exemple d'un cycle hormonal.

On citera notamment à titre d'exemple pour la détection d'une glycoprotéine ou d'un mucopolysaccharide, et par conséquent, la période fertile chez la femme, les réactifs suivants : la safranine, le bleu de toluidine O, le bleu Alcian, le bleu trypan, un sel de tolonium, le PAS (Schiff périodique acide) ou un mélange de ceux-ci, éventuellement couplés à un agent favorisant la réaction colorée, tel que le polyvinylpyrrolidone.

## Revendications

1. Dispositif (1) à usage unique, de recueil d'un liquide corporel, comprenant un moyen de collecte (3) d'un liquide corporel, s'étendant selon la longueur dudit dispositif, et un moyen anti-retour (4) du liquide corporel collecté, fermant le moyen de collecte (3) lorsque ledit liquide s'écoule dans ledit dispositif, dans le sens opposé à celui de la collecte, **caractérisé en ce que, en outre** :
- le dispositif (1) comprend un élément (2) de conduit rigide, adapté en forme et dimensions pour être logé et retenu directement au contact avec la paroi intérieure d'une cavité intracorporelle allongée dans la cavité intracorporelle, présentant une extrémité ouverte ou col (2a) et une extrémité fermée ou fond (2b), suffisamment raide selon sa longueur pour être poussé par son fond, et introduit par son col dans ladite cavité intracorporelle, et dont la surface extérieure est biocompatible avec la paroi intérieure de ladite cavité intracorporelle;
- le moyen de collecte (3) du liquide corporel est monté ou intégré à l'intérieur de l'élément de conduit (2), et s'étend d'une entrée (3a) de section transversale relativement importante, en vis-à-vis du col (2a) de l'élément de conduit (2), à une sortie (3b) de section transversale relativement faible, en vis-à-vis du fond (2b) de l'élément de conduit;
- le moyen (4) anti-retour du liquide corporel collecté ferme la sortie (3b) de section transversale relativement faible du moyen de collecte (3) lorsque ledit liquide s'écoule dans l'élément de conduit (2), dans le sens opposé à celui de la collecte.

2. Dispositif selon la revendication 1, caractérisé en ce que l'élément de conduit (2) comprend un tube (5) relativement rigide avec un col (5a) et un fond (5b), et éventuellement un manchon (6) biocompatible entourant au moins la paroi latérale dudit tube.

3. Dispositif selon la revendication 1, caractérisé en ce que le moyen de collecte (3) avec son moyen (4) anti-retour sont montés de manière amovible par rapport à l'élément de conduit (2).

4. Dispositif selon la revendication 1, caractérisé en ce que le moyen anti-retour (4) comprend un dispositif de clapet (7) monté de manière mobile par rapport à la sortie (3b) du moyen de collecte (3), rappelé contre ladite sortie dans le sens opposé à celui de la collecte du fluide corporel, éventuellement disposé dans un collecteur (8) dudit fluide.

5. Dispositif selon la revendication 1, caractérisé en ce qu'un lien d'extraction (15) est fixé à l'élément de conduit (2), du côté de son fond (2b), éventuellement dans une gorge (5c).

6. Dispositif selon la revendication 1, caractérisé en ce qu'un moyen (9) réactionnel est disposé à l'intérieur de l'élément de conduit (2), du côté de son fond (26), en vis-à-vis de la sortie (3b) du moyen de collecte (3), et comprend au moins un réactif, éventuellement déposé sur une couche absorbante, susceptible de réagir avec au moins un composant du liquide corporel, ou en présence d'une condition biologique ou biochimique dudit liquide, pour donner au moins un produit de réaction, notamment coloré, révélant la présence de ladite condition biologique ou biochimique, ou dudit composant dans ledit liquide corporel.

7. Dispositif selon la revendication 6, caractérisé en ce que le produit de réaction est coloré, le fond (2b) est transparent (10), et le réactif (9) est disposé en couche relativement mince contre le fond (2b), en vis-à-vis de la sortie (3b) du moyen de collecte.

8. Dispositif selon la revendication 7, caractérisé en ce que la couche de réactif (9) est recouverte par un voile (11) hemi-perméable, perméable vis-à-vis du liquide corporel, dans le sens de la collecte, et retenant ledit liquide dans l'autre sens, et en ce que le voile ou la couche ou les deux sont éventuellement imprégnés d'un agent fluidifiant favorisant le passage des composés à détecter.

9. Dispositif selon la revendication 1, caractérisé en ce que la forme et les dimensions de l'élément (2) de conduit sont adaptées à celles d'une cavité vaginale, et le liquide corporel comprend de la glaire cervicale.

10. Dispositif selon la revendication 9, caractérisé en ce que le composant de la gare cervicale dont on recherche la présence est un composant peroxydasique, et le réactif est un composé d'oxydoréduction, dont au moins la forme oxydée est colorée, par exemple le gaïacol.

11. Dispositif selon la revendication 9, caractérisé en ce que le composant de la glaire cervicale dont on recherche la présence est un mucopolysaccharide ou une glycoprotéine, et le réactif est choisi parmi la safranine, le bleu de toluidine O, le bleu Alcian, le bleu trypan, un sel de tolonium, le PAS (Schiff périodique acide) ou un mélange de ceux-ci.

12. Ensemble prêt à l'emploi, comprenant un dispositif (1) selon la revendication 1 et système applicateur (16).

13. Ensemble selon la revendication 12, caractérisé en ce que le système applicateur (16) comprend un tube-guide (13) à l'intérieur duquel le dispositif (1) de recueil est inséré, et un poussoir (14) logé à l'intérieur du tube-guide, en butée contre le dispositif de recueil.

## Claims

1. Disposable device (1) for recovery of a body fluid, comprising a collection means (3) for collecting a body fluid, extending along the length of the said device, and a nonreturn means (4) for the body fluid which has been collected, closing the collection means (3) when the said fluid flows into the said device, in the direction counter to that of collection, characterized in that, furthermore:
- the device (1) comprises a relatively rigid conduit element (2), adapted in shape and dimensions to be fitted and held directly in contact with the inner wall of an elongate intra-corporeal cavity in the intracorporeal cavity, having an open end or neck (2a), and a closed end or base (2b), sufficiently stiff along its length to be pushed via its base, and introduced via its neck, into the said intracorporeal cavity, and the outer surface of which is biocompatible with the inner wall of the said intracorporeal cavity;
- the collection means (3) for collecting the body fluid is mounted or integrated inside the conduit element (2), and extends from an inlet (3a) of relatively large cross-section, facing the neck (2a) of the conduit element (2), to an outlet (3b) of relatively small cross-section, facing the base (2b) of the conduit element;
- the nonreturn means for the body fluid which has been collected closes the outlet (3b) of relatively small cross-section of the collection means (3) when the said fluid flows into the conduit element (2), in the direction counter to that of collection.

2. Device according to Claim 1, characterized in that the conduit element (2) comprises a relatively rigid tube (5) with a neck (5a) and a base (5b), and if appropriate a biocompatible sleeve (6) surrounding at least the side wall of the said tube.

3. Device according to Claim 1, characterized in that the collection means (3) and its nonreturn means (4) are mounted in a removable manner in relation to the conduit element (2).

4. Device according to Claim 1, characterized in that the nonreturn means (4) comprises a valve device (7) mounted movably with respect to the outlet (3b) of the collection means (3), held against the said outlet in the direction counter to that of the collection of the body fluid, if appropriate arranged in a collector (8) for the said fluid.

5. Device according to Claim 1, characterized in that a removal line (15) is fixed to the conduit element (2), on the side of its base (2b), if appropriate in a groove (5c).

6. Device according to Claim 1, characterized in that a reaction means (9) is arranged inside the conduit element (2), at its base (2b), facing the outlet (3b) of the collection means (3), and comprises at least one reagent, if appropriate deposited on an absorbent layer, capable of reacting with at least one component of the body fluid, or in the presence of a biological or biochemical state of the said fluid, in order to give at least one reaction product, especially coloured, revealing the presence of the said biological or biochemical state, or of the said component in the said body fluid.

7. Device according to Claim 6, characterized in that the reaction product is coloured, the base (2b) is transparent (10), and the reagent (9) is arranged in a relatively thin layer against the base (2b), facing the outlet (3b) of the collection means.

8. Device according to Claim 7, characterized in that the layer of reagent (9) is covered by a semipermeable web (11), which is permeable with respect to the body fluid in the direction of collection, and holds back the said fluid in the other direction, and in that the web or the layer or both is/are impregnated if appropriate with a fluidizing agent promoting the passage of the compounds which are to be detected.

9. Device according to Claim 1, characterized in that the shape and the dimensions of the conduit element (12) are adapted to those of a vaginal cavity, and the body fluid comprises the cervical mucus.

10. Device according to Claim 9, characterized in that the component of the cervical mucus whose presence is being tested for is a peroxidase component, and the reagent is an oxidation-reduction compound, of which at least the oxidized form is coloured, for example guaiacol.

11. Device according to Claim 9, characterized in that the component of the cervical mucus whose presence is being tested for is a mucopolysaccharide or a glycoprotein, and the reagent is chosen from among safranin, toluidine blue O, Alcian blue, trypan blue, a tolonium salt, PAS (periodic acid-Schiff), or a mixture of these.

12. Assembly ready for use and comprising a device (1) according to Claim 1 and an applicator system (16).

13. Assembly according to Claim 12, characterized in that the applicator system (16) comprises a guide tube (13) inside which the recovery device (1) is inserted, and a pusher (14) housed inside the guide tube, abutting against the recovery device.

## Patentansprüche

1. Vorrichtung (1) zur einmaligen Verwendung zum Sammeln einer Körperflüssigkeit, mit einem Mittel (3) zum Sammeln einer Körperflüssigkeit, das sich längs der Vorrichtung erstreckt, und mit einem Mittel (4) gegen das Zurückfließen der gesammelten Körperflüssigkeit, das das Mittel (3) zum Sammeln verschließt, wenn die Flüssigkeit in einer der Sammelrichtung entgegengesetzten Richtung in die Vorrichtung fließt, dadurch gekennzeichnet, daß außerdem folgendes gilt:
- die Vorrichtung (1) weist ein starres Führungselement (2) auf, das in seiner Form und in seinen Abmessungen daran angepaßt ist, direkt im Kontakt mit der inneren Wand einer langgestreckten Körperhöhle in der Körperhöhle aufgenommen und zurückgehalten zu werden, wobei das Führungselement (2) ein offenes Ende oder einen Hals (2a) und ein geschlossenes Ende oder einen Boden (2b) aufweist und entlang seiner Länge hinreichend starr ist, um an seinem Boden geschoben und mit seinem Hals in die Körperhöhle eingeführt zu werden, und dessen äußere Oberfläche mit der inneren Wand der Körperhöhle biologisch verträglich ist;
- das Mittel (3) zum Sammeln der Körperflüssigkeit wird im Inneren des Führungselements (2) angeordnet oder in dieses integriert und erstreckt sich von einem Einlaß (3a) mit relativ großem Querschnitt benachbart zum Hals (2a) des Führungselements (2) bis zu einem Auslaß (3b) mit relativ geringem Querschnitt benachbart zum Boden (2b) des Führungselements;
- das Mittel (4) gegen das Zurückfließen der gesammelten Körperflüssigkeit verschließt den Auslaß (3b) mit relativ geringem Querschnitt des Mittels (3) zum Sammeln dann, wenn diese Flüssigkeit in der der Sammelrichtung entgegengesetzten Richtung in das Führungselement fließt.

2. Vorrichtung nach Anspruch 1, dadurch gekennzeichnet, daß das Führungselement (2) eine relativ starre Röhre (5) mit einem Hals (5a) und einem Boden (5b), und gegebenenfalls eine biologisch verträgliche Ummantelung (6), die zumindest die Seitenwand der Röhre umschließt, aufweist.

3. Vorrichtung nach Anspruch 1, dadurch gekennzeichnet, daß das Mittel (3) zum Sammeln mit seinem Mittel (4) gegen das Zurückfließen bezüglich des Führungselements (2) unbeweglich angebracht ist.

4. Vorrichtung nach Anspruch 1, dadurch gekennzeichnet, daß das Mittel (4) gegen das Zurückfließen eine Klappenvorrichtung (7) aufweist, die bezüglich des Auslasses (3b) des Mittels (3) zum Sammeln beweglich angebracht ist, die in der dem Sammeln der Körperflüssigkeit entgegengesetzten Richtung auf den Auslaß hin verschiebbar ist, und die gegebenenfalls in einem Sammelgefäß (8) für die Flüssigkeit angeordnet ist.

5. Vorrichtung nach Anspruch 1, dadurch gekennzeichnet, daß am Führungselement (2) auf seiten dessen Bodens (2b) ein Abziehband (15), gegebenenfalls in einer Kehlnut (5c), befestigt ist.

6. Vorrichtung nach Anspruch 1, dadurch gekennzeichnet, daß ein Reaktionsmittel (9) im Inneren des Führungselements (2) auf seiten dessen Bodens (2b) benachbart zum Auslaß (3b) des Mittels (3) zum Sammeln angeordnet ist, das zumindest ein gegebenenfalls auf einer absorbierenden Schicht aufgebrachtes Reagenz aufweist, das dazu fähig ist, mit zumindest einem Bestandteil der Körperflüssigkeit oder bei Vorhandensein eines biologischen oder biochemischen Zustands der Flüssigkeit zu reagieren, um zumindest ein insbesondere gefärbtes Reaktionsprodukt zu ergeben, das die Anwesenheit des biologischen oder biochemischen Zustands oder des Bestandteils in der Körperflüssigkeit anzeigt.

7. Vorrichtung nach Anspruch 6, dadurch gekennzeichnet, daß das Reaktionsprodukt gefärbt ist, daß der Boden (2b) transparent (10) ist, und daß das Reagenz (9) in einer relativ dünnen Schicht am Boden (2b) benachbart zum Auslaß (3b) des Mittels zum Sammeln aufgebracht ist.

8. Vorrichtung nach Anspruch 7, dadurch gekennzeichnet, daß die Schicht an Reagenz (9) von einer semipermeablen Abdeckung (11) bedeckt ist, die für die Körperflüssigkeit in der Richtung des Sammelns durchlässig ist und die die Flüssigkeit in der anderen Richtung zurückhält, und daß die Abdeckung oder die Schicht oder beide gegebenenfalls mit einem Verflüssigungsmittel imprägniert sind, das den Durchtritt der nachzuweisenden Verbindungen begünstigt.

9. Vorrichtung nach Anspruch 1, dadurch gekennzeichnet, daß die Form und die Abmessungen des Führungselements (2) an diejenigen einer Vaginalhöhle angepaßt sind, und daß die Körperflüssigkeit Cervicalschleim aufweist.

10. Vorrichtung nach Anspruch 9, dadurch gekennzeichnet, daß der Bestandteil des Cervicalschleims, nach dessen Anwesenheit gesucht wird, eine Peroxidase-Verbindung ist, und daß das Reagenz eine Redoxverbindung ist, bei der zumindest die oxidierte Form gefärbt ist, z.B. Guajakol.

11. Vorrichtung nach Anspruch 9, dadurch gekennzeichnet, daß der Bestandteil des Cervicalschleims, nach dessen Anwesenheit gesucht wird, ein Mucopolysaccharid oder ein Glycoprotein ist, und daß das Reagenz ausgewählt ist unter Safranin, Toluidin-Blau O, Alcian-Blau, Trypan-Blau, einem Toloniumsalz, PAS (periodic acid-Schiff) oder einem Gemisch davon.

12. Gebrauchsfertiger Zusammenbau mit einer Vorrichtung (1) nach Anspruch 1 und einem Einführungssystem (16).

13. Zusammenbau nach Anspruch 12, dadurch gekennzeichnet, daß das Einführungssystem (16) eine Führungsröhre (13) aufweist, in deren Innerem die Aufnahmevorrichtung (1) eingeschoben ist, und einen Schieber (14), der im Inneren der Führungsröhre an die Aufnahmevorrichtung anstoßend angeordnet ist.
